# EUROPÄISCHE PATENTSCHRIFT

(11) **EP 0 565 978 B1**
(45) Veröffentlichungstag und Bekanntmachung des Hinweises auf die Patenterteilung: **18.06.1997**
(21) Anmeldenummer: 93105560.2
(22) Anmeldetag: 03.04.1993
(51) Int. Cl.: B01J 20/32, C12N 11/08

(54) **Aktivierte Trägermaterialien, ihre Herstellung und Verwendung**
Activated carriers, their fabrication and use
Support activés, leur fabrication et utilisation

(30) Priorität: 16.04.1992 DE 4212730
(43) Veröffentlichungstag der Anmeldung: 20.10.1993
(73) Patentinhaber: MERCK PATENT GmbH, D-64271 Darmstadt (DE)
(72) Erfinder: Müller, Egbert, Dr., W-6106 Erzhausen (DE); Badel, Kerstin, W-4052 Korschenbroich (DE); Müller, Andreas, W-6230 Frankfurt 80 (DE); Herbert, Stefan, W-6492 Sinntal 2 (DE); Seiler, Anja, W-8751 Stockstadt/Main (DE)

(56) Entgegenhaltungen:
- EP-A- 0 165 912
- EP-A- 0 337 144
- EP-A- 0 392 735
- EP-A- 0 392 783
- FR-A- 2 476 125
- US-A- 4 332 694
- J. OF CHROMATOGRAPHY Bd. 512, 1990, Seiten 345 - 363 P.COLEMAN 'IMMOBILIZATION OF PROTEIN A AT HIGH DENSITY ON AZLACTONE-FUNCTIONAL POLYMERIC BEADS AND THEIR USE IN AFFINITY CHROMATOGRAPHY'
- APPLIED BIOCHEMISTRY AND BIOTECHNOLOGY Bd. 31, Nr. 1, 1. Oktober 1991, Seiten 43 - 52 J. MORENO 'HYDROLYSIS OF NUCLEIC ACIDS IN SINGLE-CELL PROTEIN CONCENTRATES USING IMMOBILIZED BENZONASE'
- J. OF MOLECULAR CATALYSIS Bd. 69, Nr. 3, 1. November 1991, Seiten 419 - 427 J. MORENO 'CONTRIBUTION TO THE STUDY OF THE ENZYMATIC ACTIVITY OF BENZONASE'

## Beschreibung

Die Erfindung betrifft aktivierte Trägermaterialien, sowie Affinitätsträger und immobilisierte Enzyme, die aus diesen aktivierten Trägermaterialien herstellbar sind.

Bei Verfahren der Affinitätschromatographie werden spezifische Wechselwirkungen zwischen Analyten und sogenannten Affinitätsliganden zur Abtrennung von Begleitsubstanzen ausgenutzt. Die Analyten werden reversibel an die trägergebundenen Affinitätsliganden gebunden. Begleitsubstanzen werden nicht gebunden und lassen sich deshalb leicht auswaschen. Anschließend wird der Analyt unter Ausnutzung der Reversibilität der Bindung freigesetzt. Die Freisetzung erfolgt beispielsweise durch pH-Änderungen, Änderung der Ionenstärke oder durch den Zusatz von gelöstem Affinitätsliganden zum Elutionsmittel. Einzelheiten dieser Verfahren und Verfahrensvarianten sind dem Fachmann bekannt.

Zu den Verfahren der Affinitätschromatographie gehört die Reinigung von Antikörpern, die gegen ein bestimmtes Protein gerichtet sind. Das Protein wird an ein chromatographisches Trägermaterial gebunden. Die Lösung, die den zu reinigenden Antikörper enthält, wird auf das Trägermaterial aufgetragen. Die nicht-bindenden Anteile der Antikörperpräparation werden ausgewaschen und die gereinigten Antikörper anschließend eluiert. In Umkehrung dieses Verfahrens können auch bestimmte Proteine mittels Affinitätschromatographie gereinigt werden, wobei ein Antikörper, der gegen dieses Protein gerichtet ist, an das chromatographische Trägermaterial gebunden wird. Bei anderen Anwendungen der Affinitätschromatographie werden beispielsweise Coenzyme an den chromatographischen Träger gebunden und dienen zur Reinigung von Enzymen, die dieses Coenzym binden. Statt der Coenzyme können auch Farbstoffe mit ähnlichem Erfolg eingesetzt werden. Beispielhaft sind in Tabelle 1 eine Reihe von Affinitätsliganden zusammengestellt. Weitere Affinitätsliganden und Analyte, sowie Varianten von Verfahren finden sich in Handbüchern beispielsweise bei Kirk-Othmer Encyclopedia of Chemical Technology (Seite 35-40, 3^{rd} edition, 1978, John Wiley and Sons) und in Protein Purification, Janson, J.-C. und Ryden, L. (eds.) (Seite 275-325, 1989, VCH Publishers), oder auch bei Vijayalakshimi, M.A. (1989) TIBTECH 7, Seite 71-76.

**Tabelle 1**

| Affinitätsligand | Analyt (Beispiel) |
|---|---|
| Protein A | Immunglobuline |
| Concanavalin A | Glycoproteine |
| Biotin | Avidin/Streptavidin |
| Avidin | Biotin |
| Streptavidin | Biotin |
| 5'-Adenosinmonophosphat | NAD-abhängige Oxidoreduktasen |
| 2',5'-Adenosindiphosphat | NADP-abhängige Oxidoreduktasen |
| Aminoacridin | RNA oder DNA |
| Boronsäure | Katecholamine |
| Boronsäure | glykosyliertes Hämoglobin |
| Iminodiessigsäure | Metalloproteine |
| "thiophile" Liganden | Immunglobuline |
| Cibachromblau | monoklonale Antikörper |

Bei der Affinitätschromatographie werden häufig unterschiedliche Liganden benötigt. Es hat sich deswegen eingebürgert, aktivierte Trägermaterialien bereitzustellen, an die der jeweilige Ligand durch vereinfachte Verfahren gebunden werden kann. Eines der ersten Produkte war BrCN-Agarose: vernetzte Agarose, die mit Bromcyan aktiviert ist. Diese reagiert mit primären Aminogruppen des Liganden und bindet diesen somit an das Trägermaterial. Verbesserungen bezüglich der Druckstabilität der Trägermaterialien wurden beispielsweise durch die Einführung von vernetzten Poly-(meth)-acrylsäurederivaten oder durch die Verwendung von Silikagel als Basismaterial erhalten.

Nach EP 064 833 wird Silikagel mit gamma-Glycidoxypropyltrimethoxysilan umgesetzt, wobei ein aktiviertes Trägermaterial entsteht, an dessen Oxirangruppe Affinitätsliganden gebunden werden können. Nach DE 40 02 044 werden beispielsweise "thiophile" Liganden an Oxirangruppen gebunden; diese Affinitätsträger eignen sich besonders zur Reinigung von Immunglobulinen, z.B. von monoklonalen Antikörpern. US 4,737,560 beschreibt aktivierte Trägenmaterialien auf Grundlage von vernetzten Polymeren, die Azlactonverbindungen als aktive Gruppierung enthalten. Das Bindungsverhalten dieser Materialien ist jedoch unbefriedigend.

In EP 0 172 579 werden Trägermaterialien beschrieben, die einen Kern aus Silikagel besitzen, auf den ein vernetztes Polymeres aufgebracht ist. Dieses Polymere kann als aktivierte Gruppe beispielsweise die Oxirangruppe enthalten. Die Bindung des Polymeren an den Träger erfolgt durch Reaktion eines Teiles der Oxirangruppen. Dabei entstehen Si-O-Bindungen, die empfindlich gegen Hydrolyse sind. Da vorgeformte Polymere an das Silikagel gebunden werden, ist die Beladungsdichte eingeschränkt Die Vernetzung des Polymers behindert außerdem den Zutritt von hochmolekularen Verbindungen wie Proteinen oder Nukleinsäuren.

Aus EP-A-0337144 sind Pfropfpolymerisate bekannt, die unter anderem Monomere enthalten können, die geeignet sind Affinitätsliganden oder Enzyme zu binden. Diese Monomeren enthalten Carboxyl-, Amino-, Hydroxyl- oder Aldehydgruppen. Aus US-A-4332694 sind epoxidgruppenhaltige aktivierte Trägermaterialien bekannt, die Polymere enthalten, welche auf einen porösen anorganischen Träger gebunden sind. Aus EP-A-0392735, EP-A-0392783 und J.Chromat.**512**, 345-363 (1990) sind Polymere, insbesondere hoch-vernetzte Polymere, bekannt, die Azlactongruppen enthalten.

Da bei der Affinitätschromatographie der Analyt häufig eine Verbindung mit hohem, Molekulargewicht ist (> 10³), ist die Geschwindigkeit des Massentransfers und damit die Produktivität des Trennverfahrens durch Diffusionsvorgänge eingeschränkt. In US 5,019,270 werden chromatographische Träger offenbart, bei denen ausgehend von einer speziellen Geometrie der Trägerteilchen und der daraus resultierenden Flüssigkeitsdynamik ein beschleunigter Massentransfer erreicht wird. In EP 0 295 073 wird eine andere Methode für einen beschleunigten Massentransfer offenbart: Die Affinitätsliganden werden durch die Verwendung von Polyethylenglycol als Spacer besser zugänglich gemacht. Die Bindungskapazität dieser Materialien ist aber in ähnlicher Weise wie bei Materialien nach EP 0 064 833 eingeschränkt.

Aktivierte Trägermaterialien, wie sie zur Herstellung von Affinitätsträgern benutzt werden, sind weiterhin prinzipiell dazu geeignet, Enzyme zu immobilisieren. Derartige immobilisierte Enzyme weisen oft eine verbesserte Stabilität auf. Sie lassen sich außerdem leicht aus dem Reaktionsansatz entfernen und können auch wiederverwendet werden. Eine weitere Verwendung von immobilisierten Enzymen ist die Bereitstellung von Enzymreaktoren, bei denen unter Durchflußbedingungen eine kontinuierliche Reaktionsführung möglich ist. Es hat sich jedoch gezeigt, daß häufig die die Enzyme bei der Immobilisierung inaktiviert werden und die Ausbeute an gebundenem Enzym nach der Bindungsreaktion äußerst niedrig ist. Dabei sinkt insbesondere die Reaktivität gegen hochmolekulare Substrate (Molekulargewicht > 10³). Als Beispiel für derartige Enzyme, die bevorzugt hochmolekulare Substrate umsetzen, und das mit bisher bekannten Methoden nicht immobilisiert werden können, seien Benzonase, eine Nukleinsäurehydrolase, die RNA, sowie ein- und doppelsträngige DNA in kleine biologisch unwirksame Oligonukleotide hydrolysiert, sowie Proteinase K genannt. Benzonase ist geeignet, in biologisch gewonnenen pharmazeutischen Wirkstoffen, eventuell noch vorhandene Nukleinsäuren abzubauen und somit die unerwünschte Übertragung von genetischem Material auszuschließen. Ein derartiger Prozess wäre bevorzugt in einem Durchflußreaktor auszuführen. Voraussetzung dafür ist die Immobilisierung der Benzonase an einem geeigneten aktivierten Träger unter Erhalt der Reaktivität gegen hochmolekulare Nukleinsäuren.

Weitere Enzyme, bei deren Immobilisieruing ähnliche Probleme auftreten, sind dem Fachmann aus der Literatur bekannt.

Es besteht also die Aufgabe, ein aktiviertes Trägermaterial bereitzustellen, an das auf einfache Art und Weise Affinitätsliganden oder Enzyme gebunden werden können. Die resultierenden Affinitätsträger sollen eine hohe Bindungskapazität besitzen, sowie einen hohen Massentransfer erlauben, bei der Immobilisierung der Enzyme soll deren Reaktivität, insbesondere gegen hochmolekulare Substrate, erhalten bleiben.

Überraschend wurde gefunden, daß (Meth)acrylsäurederivate, die eine Oxiran- oder eine Azlactongruppe, oder die Vorstufe einer Azlactongruppe enthalten, auf Basisträger, die aliphatische Hydroxylgruppen auf ihrer Oberfläche enthalten, aufgepfropft werden können. Dabei wird erfindungsgemäß die Polymerisation mit Cer(IV)-Ionen gestartet: G.Mino und S.Kaizerman (1958) J.Polymer Science **31**, 242-243; G.Mino et al. (1959) J.Polymer Science **38**, 393-401. Die nach dieser Methode erhältlichen Trägermaterialien lassen sich auf einfache Weise zu Affinitätsträgern umsetzen und weisen eine hohe Bindungskapazität, sowie einen hohen Massentransfer auf. Hervorragende Eigenschaften weisen auch aktivierte Trägermaterialien mit einer Azlactongruppierung auf, deren Azlactongruppe über eine Thioetherbindung an den Träger gebunden ist. Diese aktivierten Trägermaterialien weisen auch dann besonders vorteilhafte Eigenschaften auf, wenn die Thioetherbindung direkt mit dem Basisträger verbunden ist.

Gegenstand der Erfindung sind somit aktivierte Trägermaterialien auf der Grundlage von hydroxylgruppenhaltigen Basisträgern, auf deren Oberflächen Polymeren kovalent gebundenen sind, dadurch gekennzeichnet, daß
a) der Basisträger aliphatische Hydroxylgruppen enthält,
b) die kovalentgebundenen Polymeren über eine endständige Monomereinheit an den Basisträger gebunden sind,
c) die Polymeren Monomereinheiten der Formel I enthalten,
d) die Monomereinheiten linear verknüpft sind, 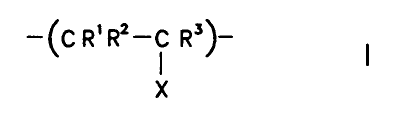 worin
   - R¹, R² und R³: unabhängig voneinander
   H oder CH₃
   und
   - X: eine aktivierte Gruppierung enthaltend eine Azlacton- oder eine Oxirangruppe
bedeuten.

Gegenstand der Erfindung sind auch aktivierte Trägermaterialien, die einen Rest entsprechend einer der Formeln VIIa oder VIIb, 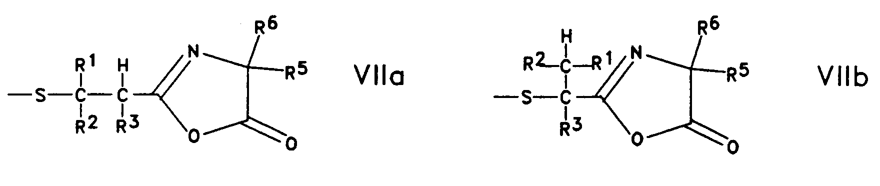 worin
- R¹, R² und R³: unabhängig voneinander
H oder CH₃
und
- R⁵ und R⁶: jeweils unabhängig von einander
H oder Alkyl mit 1-5 C-Atomen
bedeuten, enthalten.

Gegenstand der Erfindung sind Verfahren zur Herstellung von aktivierten Trägermaterialien ausgehend von hydroxylgruppenhaltigen Basisträgern, auf deren Oberflächen Polymere durch Pfropfpolymerisation kovalent gebunden werden, dadurch gekennzeichnet, daß die hydroxylgruppenhaltigen Basisträgerteilchen in Gegenwart von Cer-IV)-Ionen in einer Lösung enthaltend Monomere der Formel IV 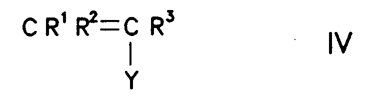 worin
- R¹, R² und R³: unabhängig voneinander
H oder CH₃
und
- Y: eine aktivierte oder aktivierbare Gruppierung, enthaltend eine Oxiran- oder eine Azlactongruppe oder einen Rest, aus dem eine Azlactongruppe hervorgehen kann,
bedeuten,
suspendiert und polymerisiert werden, und gegebenenfalls eine Vorläuferverbindung in ein Azlactonringsystem umgewandelt wird.

Gegenstand der Erfindung sind auch Verfahren zur Herstellung von aktivierten Trägermaterialien ausgehend von Basisträgern, die Thiolgruppen enthalten, durch Umsetzung mit einem Vinylazlacton der Formel VIII 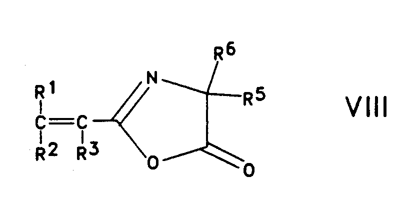 worin
- R¹, R² und R³: unabhängig voneinander
H oder CH₃
und
- R⁵ und R⁶: jeweils unabhängig von einander
H oder Alkyl mit 1-5 C-Atomen
bedeuten.

Gegenstand der Erfindung ist die Verwendung der erfindungsgemäßen aktivierten Trägermaterialien für die Bindung von Affinitätsliganden und für die Immobilisierung von Enzymen.

Gegenstand der Erfindung sind auch Verfahren zur Herstellung von Affinitätsträgern, dadurch gekennzeichnet, daß Affinitätsliganden, die im einzelnen dem Fachmann bekannt sind, an aktivierte Trägermaterialien entsprechend der vorliegenden Erfindung gebunden werden.

Gegenstand der Erfindung sind auch Affinitätsträger herstellbar durch Bindung von Affinitätsliganden an die erfindungsgemäßen aktivierten Trägermaterialien.

Gegenstand der Erfindung ist weiterhin die Verwendung von Affinitätsträgern entsprechend der vorliegenden Erfindung bei der Aufreinigung von Biopolymeren.

Gegenstand der Erfindung sind auch Verfahren zur Immobilisierung von Enzymen, dadurch gekennzeichnet, daß Enzyme an aktivierte Trägermaterialien entsprechend der vorliegenden Erfindung gebunden werden.

Gegenstand der Erfindung sind auch immobilisierte Enzyme herstellbar durch Immobilisierung von Enzymen an den erfindungsgemäßen aktivierten Trägermaterialien, sowie deren Verwendung für enzymatische Umsetzungen.

Die Struktur der erfindungsgemäßen Trägermaterialien ergibt sich aus der Reaktionsfolge, die ursprünglich von Mino et al. beschrieben wurde: Die primären oder sekundären Hydroxylgruppen des Basisträgermaterials werden durch Umsetzung mit Cer(IV)-Salzen in stark saurer wäßriger Lösung zu Kohlenstoff- oder möglicherweise auch Sauerstoffradikalen umgesetzt. An diesen Radikalen setzt eine Radikalkettenreaktion ein, wobei die zugesetzten Monomeren in die Kette eingebaut werden. Diese Kette ist linear und mit einer Monomereinheit mit dem aliphatischen Rest des Basisträgers verknüpft. Die Polymerisation wird Abbruchsreaktionen unter Beteilung der Cer(IV)-Salze beendet. Deswegen ist die (mittlere) Kettenlänge durch die Konzentrationsverhältnisse des Basisträgers, des Initiators und der Monomeren beeinflußbar. Es können einheitliche Monomere oder auch Gemische verschiedener Monomere eingesetzt werden; im letzteren Fall entstehen gepfropfte Copolymerisate.

Für die Herstellung der aktivierten Trägermaterialien mit einer Azlactongruppierung, deren Azlactongruppe über eine Thioetherbindung an den Träger gebunden ist, wird folgende Reaktionsfolge benutzt:
a) Soweit der Basisträger weder Thiol- noch Oxiranreste besitzt, so werden zunächst Oxiranreste eingeführt. Dazu werden entweder aliphatische Hydroxylgruppen des Basisträgers mit Epichlorhydrin umgesetzt oder auf aliphatische Hydroxylgruppen des Basisträgers (Meth)Acrylglycidylester aufgepfropft. Durch Reaktion der Oxiranreste mit Natriumhydrogensulfid im alkalischen Medium werden anschließend nach bekannten Verfahren Thiolgruppen eingeführt.
b) Die im ersten Schritt eingeführten, oder bereits vorher vorhandenen Thiolgruppen des Basisträgers werden anschließend an die Vinyldoppelbindung eines Vinylazlactonderivates addiert. Diese Reaktion wird bevorzugtermaßen durch 1,8-Diazabicyclo[5,4,0]undec-7-en entsprechend EP 0 473 457 katalysiert. Als Vinylazlactonderivat wird Vinyldimethylazlacton besonders bevorzugt.

Basisträger im Sinne der vorliegenden Erfindung sind Partikel, auf die Polymere aufgepfropft werden. Als Basisträger können allgemein übliche poröse oder unporöse Trägerteilchen eingesetzt werden, soweit sie primäre oder sekundäre aliphatische Hydroxylgruppen an ihrer Oberfläche aufweisen. Geeignet sind unter anderem Polysaccharide auf Agarose-Basis, Cellulose, Cellulosederivate und Polymere auf Dextran-Basis. Bevorzugt sind Polymere auf Polyvinylalkohol-Basis oder Copolymere aus (Meth)acrylatderivaten und Comonomeren mit aliphatischen Hydroxylgruppen. Insbesondere sind diolmodifizierte Kieselgele als Basisträger bevorzugt. Bevorzugte, kommerziell erhältliche Basisträger sind Fractogel^{R} TSK HW 65 (S) (Fa. E. Merck), ein poröses Mischpolymerisat auf Vinylbasis, das aliphatische Hydroxylgruppen enthält (1 mÄq OH/g), und LiChrospher^{R} DIOL (Fa. E. Merck), ein diolsubstituiertes Kieselgel, ebenfalls mit aliphatischen Hydroxylgruppen.

Basisträger im weiteren Sinne sind aber auch membran- oder fadenförmige, sowie netz- oder gewebeähnliche Materialien, die aliphatische Hydroxylgruppen enthalten, oder in die nach an sich bekannten Verfahren aliphatische Hydroxylgruppen eingeführt werden können. Diese Materialien können auch zusätzlich Stützelemente zur Verbesserung ihrer mechanischen Stabilität enthalten. Unter Verwendung ihrer aliphatischen Hydroxylgruppen können diese Materialien ebenfalls zu aktivierten Trägermaterialien umgesetzt werden. Dabei werden im wesentlichen dieselben Reaktionsfolgen angewandt, wie sie im folgenden für partikuläre Basisträger beschrieben werden.

Affinitätsliganden sind chemische Verbindungen, die geeignet sind, spezifische Wechselwirkungen einzugehen, und die somit für die Verwendung in der Affinitätschromatographie geeignet sind. Beispiele für derartige Affinitätsliganden sind in Tabelle 1 zusammengestellt.

Aktivierte Gruppen im Sinne der Erfindung sind chemisch reaktionsfähige Reste, die kovalent an die Monomeren oder die Monomereinheiten von Polymeren gebunden vorliegen, und die mit dem Affinitätsliganden kovalente Bindungen eingehen. Aktivierte Gruppen im Sinne der Erfindung sind insbesondere Reste nach Formel II, die eine Oxirangruppe enthalten, und Reste nach Formel III die eine Azlactongruppe enthalten. 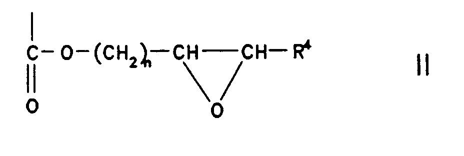 In Formel II bedeuten
- R⁴: H, Alkyl mit 1-5 C-Atomen oder Aryl mit 6-12 C-Atomen
und
- n: eine ganze Zahl zwischen 1 und 5.

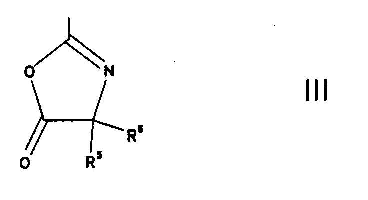

In Formel III bedeuten
- R⁵ und R⁶: unabhängig voneinander
H oder Alkyl mit 1-5 C-Atomen.

Aktivierte Trägermaterialien sind Basisträger, an die Polymere mit aktivierten Gruppen gebunden vorliegen. Affinitätsträger sind Basisträger, an die Polymere mit Affinitätsliganden gebunden sind, wobei deren Bindung mit Hilfe der aktivierten Gruppen erfolgte.

Enzyme sind Proteine, die als Biokatalysatoren wirken. Es sind jedoch auch Nukleinsäurederivate bekannt, die katalytische Funktionen besitzen (sogenannte Ribozyme), und die erfindungsgemäß ebenfalls unter dem Begriff "Enzyme" subsummiert werden. Enzyme katalysieren spezifisch bestimmte Umsetzungen, z.B. Redox-Reaktionen oder Hydrolysen, bei denen bestimmte Substrate, beispielsweise Alkohole oder Nukleinsäuren, umgesetzt werden. Häufig werden von den Enzymen bei diesen Umsetzungen Cofaktoren benötigt. Im Stoffwechsel wirken häufig mehrere Enzyme zusammen, um Reaktionsketten zu katalysieren. Einzelheiten von enzymatisch katalysierten Reaktionen, den dabei beteiligten Enzymen und Cofaktoren, sowie von biochemischen Reaktionsketten sind dem Fachmann bekannt. Es ist weiterhin bekannt, daß Enzyme auf Trägermaterialien mittels verschiedener Reaktanden, z.B. Oxiran-, Carbonylimidazol- oder Tresylderivaten, immobilisiert werden können. Wirken in einer Reaktionskette verschiedene Enzyme zusammen, so können diese auch in enger Nachbarschaft auf einem Träger immobilisiert werden, um für Zwischenprodukte die Diffusionswege kurz zu halten. Erfindungsgemäß können deswegen Enzyme einzeln immobilisiert werden, aber auch verschiedene, beispielsweise in Reaktionsketten kooperierende, Enzyme gemeinsam immobilisiert werden.

Aktivierbare Gruppen im Sinne der vorliegenden Erfindung sind Reste, die Vorläuferverbindungen für aktivierte Gruppen enthalten; als Beispiel seien Carboxylatderivate nach Formel VI genannt, die sich in Azlactongruppen nach Formel III überführen lassen. 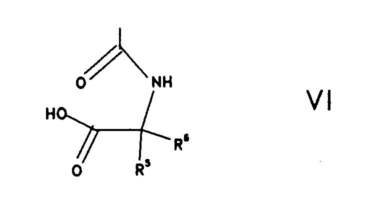 In Formel VI bedeuten
- R⁵ und R⁶: unabhängig voneinander
H oder Alkyl mit 1-5 C-Atomen.

R¹ und R² in den Formeln I, IV und V bedeuten bevorzugt H; d.h. Acryl- und Methacrylsäurederivate sind bevorzugt.

In Formel II ist n bevorzugt gleich 1, 2 oder 3 und R⁴ bedeutet bevorzugt H, Methyl, Ethyl oder Propyl. Besonders bevorzugt ist der (2,3-Epoxypropyl)-Rest als Verbindung der Formel II; d.h. n = 1 und R⁴ = H.

In den Formeln III, VI, VIIa, VIIb und VIII sind die Reste R⁵ und R⁶ bevorzugt gleich, d.h. optisch-inaktive Verbindungen sind bevorzugt. Die Reste R⁵ und R⁶ bedeuten bevorzugt H, Methyl, Ethyl oder Propyl. Besonders bevorzugt bedeuten beide Reste entweder H oder Methyl; d.h. die Derivate des Glycins und des 2-Methylalanins, sowie die zugehörigen Azlactone sind besonders bevorzugt.

Bevorzugt sind die Vinylgruppen der verwendeten Vinylazlacton-derivate nicht substituiert, d.h. R¹, R² und R³ in den Formeln VIIa, VIIb und VIII bedeuten bevorzugt H.

In besonders gelagerten Fällen kann es nützlich sein, die mögliche Beladungsdichte der Trägermaterialien zu begrenzen. Dazu werden bevorzugt neben den Monomeren nach Formel I zusätzlich Monomere nach Formel V, die keine aktivierten Gruppen enthalten, eingesetzt.

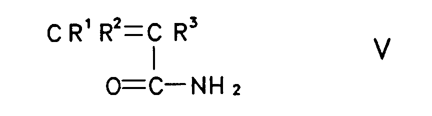

In Formel V besitzen R¹, R² und R³ dieselben Bedeutungen wie in Formel I. Bei der Herstellung der Copolymerisate werden bevorzugt 60-99,5 % Monomere nach Formel IV und 0,5 - 40 % Monomere nach Formel V eingesetzt.

Die Monomeren nach den Formeln IV und V sind, soweit sie nicht kommerziell erhältlich sind, nach dem Fachmann bekannten Standardverfahren zugänglich: Beispiele für derartige Standardverfahren sind die Acylierung nach Schotten-Baumann und die Synthese nach Strecker.

Einzelheiten dieser Verfahren sind in gängigen Handbüchern, z.B. Houben-Weyl, Methoden der Organischen Chemie, Georg Thieme Verlag, Stuttgart, beschrieben.

Die Pfropfpolymerisation nach Mino und Kaizerman wird nach bekannten Verfahren in rein wäßriger, salpetersaurer Lösung ausgeführt. Deswegen kann diese Reaktion nur mit gut wasserlöslichen Monomeren ausgeführt werden. Es zeigte sich jedoch überraschenderweise, daß die Umsetzung mit Cer(IV)-salzen auch möglich ist, wenn als Lösungsmittel eine Mischung aus Wasser und organischen Lösungsmitteln, die keine Hydroxylgruppen enthalten, benutzt wird. Besonders bevorzugt sind dabei Dioxan oder Tetrahydrofuran. Der Anteil des organischen Lösungsmittels im Reaktionsansatz beträgt bevorzugt 10-80 Volumen-%, besonders bevorzugt 20-50 Volumen-%.

Da bekanntermaßen Oxirangruppen durch Behandlung mit verdünnten Mineralsäuren hydrolysierbar sind, ist es überraschend, daß Monomere, die Oxirangruppen enthalten, mittels Cer(IV)-Salzen in mineralsaurer Lösung polymerisierbar sind und die Oxiranstruktur erhalten bleibt.

Die Vorläuferverbindungen für Azlactongruppen nach Formel VI, die in Pfropfpolymerisaten enthalten sind, werden nach dem Fachmann bekannten Verfahren in Azlactongruppen überführt. Bevorzugt für diesen Zweck ist die Umsetzung mit Essigsäureanhydrid.

Nach der Herstellung der Affinitätsträger verbleiben im allgemeinen noch aktivierte Gruppierungen, die nicht reagiert haben. Üblicherweise werden diese Gruppen durch Umsetzung mit einem Überschuß einer wasserlöslichen Amino- oder Thiolverbindung umgesetzt. Bevorzugt wird für diesen Zweck Aminoethanol benutzt. Falls der Affinitätsligand hydrolysestabil ist, können die überschüssigen aktivierten Gruppierungen auch hydrolytisch mittels verdünnter sauerstoffhaltiger Mineralsäuren, wie z.B. Schwefel- oder Salpetersäure, entfernt werden. Bevorzugt ist dabei eine Umsetzung (Dauer: 1-5 Stunden) mit 0,5 M Schwefelsäure bei 35-60 ^{o}C.

Es hat sich gezeigt, daß aus den erfindungsgemäßen aktivierten Trägermaterialien Affinitätsträger herstellbar sind, die eine höhere Bindungskapazität als übliche Trägermaterialien besitzen. Auch ist der Massentransfer und somit die Produktivität der Affinitätsträger verbessert. Diese Vorteile beruhen offensichtlich auf der Bereitstellung einer Vielzahl von aktivierten Gruppen an dem aktivierten Trägermaterial, wobei diese Gruppen durch flexible lineare Polymere verbunden sind. Bei der Herstellung von Affinitätsträgern reagieren die aktivierten Gruppen, die sterisch besonders günstig liegen. Weiterhin sind diese Affinitätsträger beständiger gegen Säuren- oder Baseneinwirkung als herkömmliche, nicht-polymerbeschichtete Trägermaterialien. Auch immobilisierte Enzyme, die aus den erfindungsgemäßen aktivierten Trägermaterialien herstellbar sind, weisen verbesserte Eigenschaften auf, wenn man diese mit Enzympräparationen vergleicht, die auf herkömmlichen Trägermaterialien immobilisiert wurden.

Die folgenden Beispiele dienen der Erläuterung der Erfindung und stellen keine Begrenzung der Erfindung dar.

### Herstellungsbeispiele

In den folgenden Herstellungsbeispielen bedeutet Raumtemperatur (RT) 15-30^{o}C. Die Polymeriation wird in einem Dreihalskolben geeigneter Größe, der mit Rührer, Tropftrichter und Thermometer ausgerüstet ist, ausgeführt. Gewaschen wird durch Absaugen auf einer Nutsche.

### Beispiel 1: Herstellung eines oxiran-aktivierten Trägers ausgehend von Fractogel^{R_}TSK HW 65(S)

Zu einer Suspension aus 50 ml sedimentiertem Fractogel^{R_}TSK HW 65 (S) und 25 ml Wasser werden mit 2 g Ammoniumcer(IV)nitrat (gelöst in 25 ml 2 M HNO₃) bei RT unter starkem Rühren vermischt. Nach 1 Minute erfolgt die Zugabe einer Lösung von 3 g (2,3-Epoxypropyl)methacrylat in 30 ml Dioxan. Es wird 3 Stunden weitergerührt. Anschließend wird die Reaktionssuspension erst mit destilliertem Wasser und dann mit 0.05 M EDTA-Lösung gewaschen.

### Beispiel 2: Herstellung eines oxiran-aktivierten Trägers ausgehend von LiChrospher^{R_}Diol

Die Herstellung erfolgt entsprechend Beispiel 1, LiChrospher^{R_}DIOL (Partikelgröße 15-25 µm, Porengröße 80 nm) wird als Basisträger anstelle von Fractogel^{R_}TSK HW 65 (S) verwendet.

### Beispiel 3: Herstellung eines Affinitätsträgers für die Metall-Chelat-Chromatographie auf Fractogel^{R_}Basis

Zu 100 ml abgesaugtem oxiran-aktiviertem Trägermaterial hergestellt nach Beispiel 1 werden 200 ml einer 0.4 M Lösung des Dinatriumsalzes der Iminodiessigsäure gegeben (pH=11). Die Lösung wird bei 45 ^{o}C 24 Stunden gerührt. Das Reaktionsprodukt wird abgenutscht, mit Wasser gewaschen und die nicht umgesetzten Oxirangruppen durch Behandlung mit 200 ml 0.5 M Schwefelsäure (2 Stunden, 45 ^{o}C) hydrolysiert.

Anschließend wird das Affinitätsträgermaterial mit 0.2 M Sulfitlösung (pH=2), sowie mit 1 M Salpetersäure und 0.5 M Natronlauge gewaschen.

### Beispiel 4: Herstellung eines Affinitätsträgers für die Metall-Chelat-Chromatographie auf LiChrospher^{R_}Diol-Basis

Zu 100 ml abgesaugtem oxiran-aktiviertem Trägermaterial hergestellt nach Beispiel 2 werden 200 ml einer 0.4 M Lösung des Dinatriumsalzes der Iminodiessigsäure gegeben (pH=9). Die Lösung wird bei 45 ^{o}C 24 Stunden gerührt. Das Reaktionsprodukt wird abgenutscht und die nicht umgesetzten Oxirangruppen durch Behandlung mit 200 ml 0.5 M Schwefelsäure (2 Stunden, 45 ^{o}C) hydrolysiert.

Anschließend wird das Affinitätsträgermaterial mit 0.2 M Sulfitlösung (pH=2) und mit 1 M Salpetersäure gewaschen. Schließlich wird mit Na-Phosphatpuffer (0.1 M; pH 7) neutral gewaschen.

### Beispiel 5: Herstellung eines Affinitätsträgers für die "thiophile" Proteinadsorption basierend auf Fractogel^{R}-TSK HW 65 (S)

Stufe 1:
   100 ml abgesaugtes oxiran-aktiviertes Trägermaterial hergestellt nach Beispiel 1 werden in 200 ml 4 M NaHS-Lösung (pH=11) suspendiert und 1 Stunde bei Raumtemperatur gerührt. Danach wird mit destilliertem Wasser gewaschen.
Stufe 2:
   Das in Stufe 1 erhaltene Material wird mit 200 ml einer 0.4 M Divinylsulfonlösung (gelöst in einer 0.5 M Na₂CO₃-Lösung; pH=11) versetzt und eine Stunde bei Raumtemperatur gerührt. Das Gel wird anschließend mit destilliertem Wasser gewaschen.
Stufe 3:
   Das aus Stufe 2 erhaltene Material wird 45 min in 200 ml 2.3 M Mercaptoethanol in 0.5 M Na₂CO₃-Lösung (pH=11) gerührt.
   Anschließend wird je zweimal mit je 200 ml 0.2 M Sulfitlösung (pH=1), 1 M Salpetersäure und 0.05 M NaOH gewaschen.

### Beispiel 6: Herstellung eines Affinitätsträgers für die "thiophile" Proteinadsorption basierend auf LiChrospher^{R_}DIOL

Stufe 1:
   100 ml abgesaugtes oxiran-aktiviertes Trägermaterial hergestellt nach Beispiel 2 werden in 200 ml 4 M NaHS-Lösung (pH=9) suspendiert und 1 Stunde bei Raumtemperatur gerührt. Danach wird mit destilliertem Wasser gewaschen.
Stufe 2:
   Das in Stufe 1 erhaltene Material wird mit 200 ml einer 0.4 M Divinylsulfonlösung (gelöst in einer 0.5 M Na₂CO₃-Lösung; pH=9) versetzt und eine Stunde bei Raumtemperatur gerührt. Das Gel wird anschließend mit destilliertem Wasser gewaschen.
Stufe 3:
   Das aus Stufe 2 erhaltene Material wird 45 min in 200 ml 2.3 M Mercaptoethanol in 0.5 M Na₂CO₃-Lösung (pH=9) gerührt.
   Anschließend wird je zweimal mit je 0.2 M Sulfitlösung (pH=1), 1 M Salpetersäure gewaschen. Schließlich wird mit Na-Phosphatpuffer (0.1 M; pH 7) neutral gewaschen.

### Beispiel 7: Herstellung eines azlacton-aktivierten Trägers ausgehend von Fractogel^{R_}TSK HW 65 (S)

Stufe 1: Pfropfpolymeres enthaltend Poly-(acryloylmethylalanin) Für die Umsetzung wird das Ausgangsprodukt suspendiert und eine Monomer- und eine Initiatorlösung vorbereitet.
Monomerlösung: 5 g Acryloyl-2-methylalanin werden in 20 ml Wasser unter Zugabe von 4 M NaOH gelöst. Nach Zugabe von 50 ml Dioxan erwärmt man auf 45 ^{o}C. Der pH-Wert wird anschließend mit 25% HCL auf pH=1.5 eingestellt.
Initiatorlösung: 3.5 g Ammoniumcer(IV)nitrat werden in 50 ml 2 M HNO₃ gelöst.
Suspension des Ausgangsmaterials: 75 ml sedimentiertes Gel Fractogel^{R_}TSK HW 65 (S) werden in 75 ml destilliertem Wasser suspendiert und die Suspension unter starkem Rühren (ca. 200 UpM) mit 100 ml Dioxan verdünnt.
   Die Suspension des Ausgangsmaterials wird auf 45 ^{o}C erhitzt, die Apparatur evakuiert und mit Argon gespült.
   Die Initiatorlösung wird zugegeben, 1 min gewartet und dann die auf 45 ^{o}C erwärmte Monomerlösung hinzugefügt. Der Ansatz wird 3 h bei 45 ^{o}C gerührt. Nach dem Abkühlen auf Raumtemperatur wird mit 0.2 M Sulfitlösung (pH=1), 0.5 M NaOH und Aceton gewaschen. Anschließend wird das Gel 24 h bei 60 ^{o}C getrocknet.
Stufe 2: Zyklisierung zum Azlactonderivat
   Das in Stufe 1 erhaltene Produkt wird mit 500 ml Essigsäureanhydrid versetzt und 2 Stunden bei 100 ^{o}C gerührt. Es wird abgesaugt und 24 Stunden bei 60 ^{o}C im Vakuumtrockenschrank getrocknet.

### Beispiel 8: Herstellung eines azlacton-aktivierten Trägers ausgehend von LiChrospher^{R_}DIOL

Stufe 1: Pfropfpolymeres enthaltend Poly-(acryloylmethylalanin) Für die Umsetzung wird das Ausgangsprodukt suspendiert und eine Monomer- und eine Initiatorlösung vorbereitet.
Monomerlösung: 5 g Acryloyl-2-methylalanin werden in 20 ml Wasser unter Zugabe von 4 M NaOH gelöst. Nach Zugabe von 50 ml Dioxan erwärmt man auf 45 ^{o}C. Der pH-Wert wird anschließend mit 25% HCL auf pH=1.5 eingestellt.
Initiatorlösung: 3.5 g Ammoniumcer(IV)nitrat werden in 50 ml 2 M HNO₃ gelöst.
Suspension des Ausgangsmaterials: 75 ml sedimentiertes Gel LiChrospher^{R_}DIOL werden in 75 ml destilliertem Wasser suspendiert und die Suspension unter starkem Rühren (ca. 200 UpM) mit 100 ml Dioxan verdünnt.
   Die Suspension des Ausgangsmaterials wird auf 45 ^{o}C erhitzt, die Apparatur evakuiert und mit Argon gespült.
   Die Initiatorlösung wird zugegeben, 1 min gewartet und dann die auf 45 ^{o}C erwärmte Monomerlösung hinzugefügt. Der Ansatz wird 3 h bei 45 ^{o}C gerührt. Nach dem Abkühlen auf Raumtemperatur wird mit 0.2 M Sulfitlösung (pH=1) und mit Na-Phosphatpuffer (0.1 M; pH 7) gewaschen. Anschließend wird das Gel 24 h bei 60 ^{o}C getrocknet.
Stufe 2: Zyklisierung zum Azlactonderivat
   Das in Stufe 1 erhaltene Produkt wird mit 500 ml Essigsäureanhydrid versetzt und 2 Stunden bei 100 ^{o}C gerührt. Es wird abgesaugt und 24 Stunden bei 60 ^{o}C im Vakuumtrockenschrank getrocknet.

### Beispiel 9: Herstellung eines Affinitätsträgers, der Protein A enthält

20 mg Protein A werden in einer Mischung aus 10 ml 1 M Ammoniumsulfatlösung und 25 ml Na-Phosphatpuffer (20 mM; pH=7.5) gelöst. 1 g azlacton-aktiviertes Trägermaterial aus Beispiel 7 wird hinzugefügt und die Suspension 24 Stunden bei Raumtemperatur geschüttelt.

Die Suspension wird anschließend mit 50 ml wäßriger Ethanolamin-Lösung (1 M) versetzt und eine weitere Stunde geschüttelt. Danach wird abgenutscht und mit 25 mM Na-Phosphat + 0.15 M NaCl (pH=7.5) gewaschen.

Der derartig erhaltene Affinitätsträger bindet 20 mg Gamma-Globulin pro Gramm feuchtes Gel (in 50 mM TRIS-Puffer, pH 8, mit 9 g/l NaCl).

### Beispiel 10: Herstellung eines azlacton-aktivierten Trägers, dessen Azlactonreste über Thioetherbrücken gebunden sind

Stufe 1: Einführung der Epoxidgruppen
   In einer Lösung von 15 g NaOH in 100 ml Wsser werden 250 ml Fractogel^{R} HW 65 (S) (Fa. E. Merck, DE) suspendiert. Unter Rühren werden 60 ml Epichlorhydrin im Laufe von 20 Minuten zugetropft. Anschließend wird die Temperatur auf 70 ^{o}C erhöht und 90 Minuten gerührt. Anschließend laßt man die Suspension wieder auf Raumtemperatur abkühlen und saugt das Gel ab. Das Gel wird anschließend mit jeweils 200 ml gewaschen: 3x Wasser, 2x Methanol und 2x Wasser.
Stufe 2: Einführung der Thiol-Gruppen
   Das Produkt aus Stufe 1 wird in 1000 ml 0,5 M Natriumcarbonatpuffer pH 11 suspendiert, unter Rühren mit 200 g NaHS (Monohydrat, Art.Nr. 17.150 78 (Jansen Chimica, DE) versetzt und eine Stunde gerührt. Anschließend wird das Produkt abgesaugt, 3x mit 200 ml Wasser gewaschen und in 500 ml 0,5 M Schwefelsäure resuspendiert. Die Suspension wird unter Rühren auf 60^{o}C erwärmt und zwei Stunden gerührt. Das Produkt wird abgesaugt und 5x mit je 200 ml Wasser, sowie 3x mit je 200 ml Aceton gewaschen und anschließend getrocknet (60 ^{o}C; 16 Stunden).
Stufe 3: Addition der Thiolgruppen an Vinyldimethylazlacton
   7 g Vinyldimethylazlacton werden in 35 ml Dimethylformamid gelöst und 3,5 g thiolderivatisiertes Fractogel ^{R} aus Stufe 2 in der Lösung suspendiert. Es werden 600 µl 1,8-Diazabicyclo(5.4.0)undec-7-en (Art.Nr. 803282; E. Merck/DE) zugefügt und 24 Stunden bei Raumtemperatur gerührt. Das Gel wird abgesaugt, 3x mit je 50 ml Dioxan gewaschen und in einer Soxhlett-Apparatur mit 300 ml Essigsäureethylester 16 Stunden extrahiert. Das Gel wird erneut abgesaugt, 3x mit je 50 ml Dioxan, sowie 3x mit je 50 ml Aceton gewaschen und 16 Stunden bei 60^{o}C getrocknet.

### Beispiel 11: Herstellung eines azlacton-aktivierten Trägers, dessen Azlactonreste über Thioetherbrücken gebunden sind

### Lösungen:

a) Starter-Lösung:
   4 g Ammoniumcer(IV)nitrat und 7,5 g Salpetersäure (Gehalt: 65%) werden in 500 ml Wasser gelöst.
b) Monomerlösung:
   3,8 g (2,3-Epoxypropyl)-methacrylat werden in 100 ml 1,4-Dioxan gelöst.

Stufe 1: Pfropfpolymerisation
   250 ml sedimentiertes Fractogel^{R} TSK HW65(S) werden in 125 ml Wasser suspendiert. Die Starterlösung wird unter Rühren zugegeben und eine Minute gerührt. Anschließend wird die Monomerlösung unter Rühren zugegeben. Nach 3 h Rühren wird das Gel über eine Glasfilternutsche abgesaugt und mit jeweils 250 ml der nachfolgenden Waschlösungen behandelt: 2x Wasser, 1x 0,05 M Nathumphosphat-Puffer (pH 7), 1x Wasser, 1x 0,05 M EDTA-Lösung, 2x Wasser, 2x Aceton und 2x Diethylether. Das Produkt wird bei 35^{o}C im Vakuumtrockenschrank getrocknet. Die Elementaranalyse ergab: C: 53,7 %; H: 7,6 %; N: 0,4 %
Stufe 2: Einführung der Thiol-Gruppen
   Das Produkt aus Stufe 1 wird in 1000 ml 0,5 M Natriumcarbonatpuffer pH 11 suspendiert, unter Rühren mit 200 g NaHS (Monohydrat, Art.Nr. 17.150 78 (Jansen Chimica, DE) versetzt und eine Stunde gerührt. Anschließend wird das Produkt abgesaugt, 3x mit 200 ml Wasser gewaschen und in 500 ml 0,5 M Schwefelsäure resuspendiert. Die Suspension wird unter Rühren auf 60^{o}C erwärmt und zwei Stunden gerührt. Das Produkt wird abgesaugt und 5x mit je 200 ml Wasser, sowie 3x mit je 200 ml Aceton gewaschen und anschließend getrocknet (60 ^{o}C; 16 Stunden).
Stufe 3: Addition der Thiolgruppen an Vinyldimethylazlacton
   7 g Vinyldimethylazlacton werden in 35 ml Dimethylformamid gelöst und 3,5 g thiolderivatisiertes Fractogel ^{R} aus Stufe 2 in der Lösung suspendiert. Es werden 600 µl 1,8-Diazabicyclo(5.4.0)undec-7-en (Art.Nr. 803282; E. Merck/DE) zugefügt und 24 Stunden b gewaschen und in einer Soxhlett-Apparatur mit 300 mi Essigsäureethylester 16 Stunden extrahiert. Das Gel wird erneut abgesaugt, 3x mit je 50 ml Dioxan, sowie 3x mit je 50 ml Aceton gewaschen und 16 Stunden bei 60^{o}C getrocknet.

### Beispiel 12: Immobilisierung von Protein A auf einem azlacton-aktivierten Träger, dessen Azlactonreste über Thioetherbrücken gebunden sind

50 mg Protein A werden in 10 ml Puffer A (25 mM Natriumphosphat; pH 7,5; 1 M Natriumsulfat) gelöst und in dieser Lösung 2 g aclactonderivatisiertes Fractogel^{R} aus Beispiel 10 suspendiert. Auf einer Schüttelmaschine wird 24 Stunden geschüttelt. Das Gel wird anschließend abgesaugt und 3x mit je 20 ml Puffer B (25 mM Natriumphosphat; pH 9,0; 1 M Ethanolamin) gewaschen und in 50 ml Puffer B suspendiert und nochmals 5 Minuten geschüttelt. Das Gel wird erneut abgesaugt, 3x mit je 20 ml Puffer C (50 mM Tris; pH 7,4; 9 g/l NaCl) gewaschen und in 50 ml Puffer C suspendiert.

### Beispiel 13: Immobilisierung von Benzonase auf einem epoxyaktivierten Träger

### Lösungen:

a) Starterlösung:
   3 g Ammoniumcer(IV)nitrat und 3 g Salpetersäure (Gehalt: 65%) werden in 190 ml Wasser gelöst.
b) Monomerlösung:
   6 g (2,3-Epoxypropyl)-methacrylat werden in 44 ml 1,4-Dioxan gelöst.
c) Puffer I:
   25 mM Tris/HCl, 0,5 mM MgCl₂, pH 8,0; 50 Vol.-% Ethylenglykol
d) Puffer L:
   25 mM Tris, 2,5 mM CaCl₂, pH 7,5; 50 Vol.-% Ethylenglykol

Stufe 1: Pfropfen eines epoxidgruppenhaltigen Linearpolymers auf Fractogel^{R} TSK HW65(S)
   100 ml sedimentiertes Fractogel^{R} TSK HW65(S) werden in 66 ml Wasser suspendiert. Die Starterlösung wird unter Rühren zugegeben und eine Minute gerührt. Anschließend wird die Monomerlösung unter Rühren zugegeben. Nach 1 h Rühren bei 180 U/min bei Raumtemperatur wird das Gel über eine Glasfilternutsche abgesaugt und mit jeweils 150 ml der nachfolgenden Waschlösungen behandelt: 4x Wasser, 3x Aceton, 3x Wasser, 1x Schwefelsäure 10%, 2x Wasser, 1x 0,2 M Phosphat-Puffer (pH 7), 3x Wasser, 3x Aceton, 1x Diethylether. Das aktivierte Gel wird bei 35^{o}C im Vakuumtrockenschrank getrocknet.
Stufe 2: Reaktion der Benzonase mit dem aktivierten Gel
   800 µl Benzonaselösung (1800 kU/ml) werden in 20 ml Puffer I gelöst. In dieser Lösung werden 2,5 g des aktivierten Gels aus Stufe 1 suspendiert. Das Reaktionsgemisch wird 20 h bei Raumtemperatur in einem Erlenmeyerkolben geschüttelt (200 UpM). Das Gel wird über eine Glasfilternutsche abgesaugt und mit je 20 ml der nachfolgend aufgeführten Waschlösungen behandelt: 2x mit Puffer I, 1x mit Wasser, 2x mit Puffer L. Das Gel wird abgesaugt und 3x mit 20 ml frischem sterilen Puffer L gewaschen und anschließend in Puffer L bei 4 ^{o}C gelagert.

### Beispiel 14: Immobilisierung von Proteinase K auf einem epoxyaktivierten Träger

### Lösungen:

a) Starterlösung:
   15 g Ammoniumcer(IV)nitrat und 15 g Salpetersäure (Gehalt: 65%) werden in 950 ml Wasser gelöst.
b) Monomerlösung:
   30 g (2,3-Epoxypropyl)-methacrylat werden in 220 ml 1,4-Dioxan gelöst.
c) Puffer I:
   25 mM Barbiturat, 5 mM MgCl₂, pH 9,2
d) Puffer I:
   50 mM Tris, 5 mM CaCl₂, pH 7,5

Stufe 1: Pfropfen eines epoxidgruppenhaltigen Linearpolymers auf Fractogel^{R} TSK HW65(S)
   500 ml sedimentiertes Fractogel^{R} TSK HW65(S) werden in 330 ml Wasser suspendiert. Die Starterlösung wird unter Rühren zugegeben und eine Minute gerührt. Anschließend wird die Monomerlösung unter Rühren zugegeben. Nach 1 h Rühren bei 180 U/min bei Raumtemperatur wird das Gel über eine Glasfilternutsche abgesaugt und mit jeweils 500 ml der nachfolgenden Waschlösungen behandelt: 4x Wasser, 3x Aceton, 3x Wasser, 1x Schwefelsäure 10%, 2x Wasser, 2x 0,2 M Phosphat-Puffer (pH 7), 3x Wasser.
Stufe 2: Reaktion der Proteinase K mit dem aktivierten Gel
   25 g Proteinase K (Katalog Nr. 24568; Fa. E. Merck, DE) werden in 5000 ml Puffer I gelöst. In dieser Lösung wird das aktivierte Gel aus Stufe 1 suspendiert. Das Reaktionsgemisch wird 22 h bei Raumtemperatur in einem Dreihalskolben gerührt. Nach 18 h wird der pH mit Natronlauge auf 9,2 nachgestellt. Das Gel wird über eine Glasfilternutsche abgesaugt und mit je 500 ml der nachfolgend aufgeführten Waschlösungen behandelt: 2x mit Puffer I, 1x mit 5 mM CaCl₂ in Wasser, 2x mit einer wäßrigen Lösung enthaltend 1 M NaCl und 5 mM CaCl₂, 1x mit 5 mM CaCl₂ in Wasser, 2x mit einer wäßrigen Lösung enthaltend 0,1 M Natriumacetat (pH 4) und 5 mM CaCl₂, 1x mit 5 mM CaCl₂ in Wasser, 2x mit wäßriger 6 M Harnstofflösung, 3x mit 5 mM CaCl₂ in Wasser, 2x mit Puffer L. Das Gel wird abgesaugt und 3x mit 500 ml frischem sterilen Puffer L gewaschen und anschließend in Puffer L bei 4 ^{o}C gelagert.

Aus den obigen Beispielen ist ersichtlich, daß die erfindungsgemäßen aktivierten Trägermaterialien hervorragend zur Herstellung von Affinitätsträgern geeignet sind, und daß die daraus hergestellten Affinitätsträger verbesserte Eigenschaften für die Affinitätschromatographie aufweisen. Weiterhin ist ersichtlich, daß die erfindungsgemäßen aktivierten Trägermaterialien hervorragend zur Immobilisierung von Enzymen geeignet sind, wobei deren Aktivität, insbesondere gegen hochmolekulare Substrate, weitgehend erhalten bleibt.

### Anwendungsbeispiele

### Anwendungsbeispiel A: Proteinbindungskapazität von verschiedenen Metallchelat-Affinitätsträgern

### Chromatographische Bedingungen:

- Gerät:: Merck-Hitachi HPLC-Inertsystem
- Säule:: Superformance^{R} 50-10 mm (E.MERCK)
- Fluß:: 1 ml/min
- Eluent:: 20 mM Na-Phosphatpuffer (pH =7) und 1 M NaCl
- Probe:: 10 mg/ml Lysozym, gelöst im Eluenten
- Wellenlänge:: 280 nm

Die Ergebnisse sind in Tabelle 2 zusammengefaßt.

**Tabelle 2**

| **Proteinbindungskapazität von verschiedenen Metallchelat-Affinitätsträgern** | | |
|---|---|---|
| | Bindung mg Lysozym / ml Gel | |
| | herkömmlich | erfindungsgemäß |
| Vergleichsmaterial¹⁾ | 40 | --- |
| Basisträger: | | |
| Fractogel^{R}-TSK HW 65 (S) | 36 ²⁾ | 65 |
| Lichrospher^{R}-DIOL | 35 ³⁾ | 70 |

| | | |
|---|---|---|
| Erläuterungen: 1) kommerziell erhältlicher Affinitätsträger | | |
| 2) Aktivierung des Basisträgers entsprechend dem Stand der Technik mittels Epichlorhydrin | | |
| 3) Aktivierung des Basisträgers entsprechend dem Stand der Technik mittels gamma-Glycidoxypropyltrimethoxysilan | | |

### Anwendungsbeispiel B: Proteinsbindungskapazität von veschiedenen "thiophilen"-Affinitätsträgern

### Chromatographiebedingungen:

- Gerät:: Merck-Hitachi HPLC-Inertsystem
- Säule:: Superformance^{R} 50-10 mm (E.MERCK)
- Fluß:: 0.25 ml/min
- Eluent:: 20 mM Na-Phosphatpuffer (pH=7) und 0.8 M Ammoniumsulfat
- Probe:: 5 mg/ml gamma-Globulin, gelöst im Eluenten
- Wellenlänge:: 280 nm

Die Ergebnisse sind in Tabelle 3 zusammengefaßt.

**Tabelle 3**

| **Proteinbindungskapazität von verschiedenen "thiophilen"-Affinitätsträgern** | | |
|---|---|---|
| | Bindung mg gamma-Globulin / ml Gel | |
| | herkömmlich | erfindungsgemäß |
| Vergleichsmaterial 1¹⁾ | 35 | --- |
| Vergleichsmaterial 2¹⁾ | 42 | --- |
| Basisträger: | | |
| Lichrospher^{R}-DIOL | 30 ²⁾ | 52 |

| | | |
|---|---|---|
| Erläuterungen: 1) kommerziell erhältlicher Affinitätsträger auf Agarosebasis | | |
| 2) Aktivierung des Basisträgers entsprechend dem Stand der Technik mittels gamma-Glycidoxypropyltrimethoxysilan | | |

### Anwendungsbeispiel C: Trennung von humanem Serumalbumin (HSA) und humanem IgG (Immunglobulin G) an einem Protein A haltigen Affintätsträger

### Chromatographische Bedingungen:

- Gerät:: Merck-Hitachi HPLC-Inertsystem
- Säule:: Superformance^{R} 25-10 mm (E.MERCK)
- Sorbens:: Affinitätsträger basierend auf Fractogel^{R_}TSK HW 65 (S) (hergestellt nach Beispiel 9)
- Fluß:: 1 ml/min
- Eluent A:: 50 mM TRIS (pH 7.4) + NaCl (9 g/l)
- Eluent B:: 1 M Essigsäure
- Stufengradient:: nach 10 Minuten Wechsel auf Eluent B
- Probe:: 2 mg HSA und 1 mg human IgG in 200 µl Eluent A
- Wellenlänge:: 280 nm

Ergebnisse: humanes Serumalbumin eluiert in der Lösungsmittelfront nach 2 min, während humanes IgG erst durch Eluent B nach 14 min eluiert wird.

### Vergleichsbeispiel D: Chromatographische Abtrennung von humanem IgG (Immunglobulin G) aus Humanserum an verschiedenen Protein A haltigen Affinitätsträgern

Verglichen wurde das Bindungsverhalten zweier Produkte mit immobilisiertem Protein A:
a) Protein A ohne Thioethergruppen gebunden (Träger: Sepharose): Sorbens A
b) Protein A gebunden nach Beispiel 12 (Sorbens B)
Je 250 µl Gel, äquilibriert mit Puffer A (25 mM Tris/HCl; pH8,0), werden in eine Minisäule (500 µl; Fa. Mobitec/DE) gefüllt. Eine Mischung aus 250 µl Humanserum und 250 µl Puffer A werden aufgetragen und die Säule mit 2 ml Puffer A gewaschen. Anschließend wird mit 2 ml Puffer B (50 mM Glycin/HCl; pH 3,0) eluiert. Die Menge an Immunglobulin G (IgG) in der Serumprobe und im Eluat werden mittels eines immun-turbidimetrischen Tests bestimmt. Die Ergebnisse sind in Tabelle 4 zusammengefaßt.

**Tabelle 4**

| | Serum | Sorbens A | Sorbens B |
|---|---|---|---|
| IgG (mg) | 3,15 | 1,05 | 2,67 |
| % | 100 | 33 | 84 |

### Anwendungsbeispiel E: Bestimmung der Aktivität von immobilisierter Benzonase

DNA-Präparationen mit verschiedenem Molekulargewicht (Hind: Hind III Fragment von LAMDA-DNA, pBr: Plasmid pBr 322, LAMDA: DNA des Phagen LAMBDA) werden mit immobilisierter Benzonase, hergestellt nach Beispiel 13, inkubiert (Schütteln bei 37 ^{o}C) und zu verschiedenen Zeiten (1, 4, 16 min.) der Anteil von hochmolekularen Substrat im Reaktionsüberstand durch Elektrophorese auf Agarosegel (0,8%) bestimmt. Die immobilisierte Benzonase ist in Tris-Puffer pH 8,0 (50 mM Tris, 1 mM MgCl₂, 50 Vol.-% Glycerin, 0,5 Vol.-% Mercaptoethanol) suspendiert; es werden jeweils 10 µl Suspension eingesetzt. Die Benzonasesuspension wird mit 50 µl der jeweiligen DNA-Lösung versetzt und inkubiert.

| Zeit: | 1 | 4 | 16 |
|---|---|---|---|
| Substrat: | | | |
| Hind¹⁾ | -- | -- | -- |
| pBr²⁾ | -- | -- | -- |
| LAMDA³⁾ | ++ | + | - |
| Erläuterung: ++ hochmolekulare Anteile im wesentlichen vorhanden + hochmolekulare Anteile deutlich abgebaut - hochmolekulare Anteile im wesentlichen abgebaut -- keine hochmolekularen Anteile mehr nachweisbar | | | |

| | | | |
|---|---|---|---|
| 1) Bruchstücke mit 23130, 9416, 6557, 4361, 2322, 2027, 564 und 125 Basenpaaren Lange | | | |
| 2) 4361 Basenpaare | | | |
| 3) 48502 Basenpaare | | | |

## Patentansprüche

1. Aktivierte Trägermaterialien auf der Grundlage von hydroxylgruppenhaltigen Basisträgern, auf deren Oberflächen Polymere kovalent gebunden sind, dadurch gekennzeichnet, daß
a) der Basisträger aliphatische Hydroxylgruppen enthält,
b) die kovalent gebundenen Polymeren über eine endständige Monomereinheit an den Träger gebunden sind,
c) die Polymeren Monomereinheiten der Formel I enthalten,
d) die Monomereinheiten linear verknüpft sind, 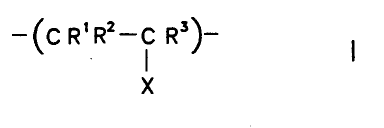 worin
R¹, R² und R³ unabhängig voneinander
H oder CH₃
und
X eine aktivierte Gruppierung enthaltend eine Azlacton- oder eine Oxirangruppe
bedeuten.

2. Aktivierte Trägermaterialien nach Anspruch 1, dadurch gekennzeichnet, daß der Rest X in Formel I einen Rest nach Formel II bedeutet, 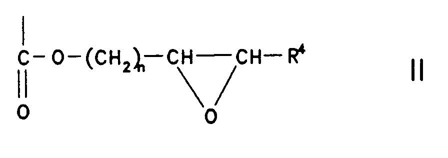 worin
R⁴ H, Alkyl mit 1-5 C-Atomen oder Aryl mit 6-12 C-Atomen
und
n eine ganze Zahl zwischen 1 und 5
bedeuten.

3. Aktivierte Trägermaterialien nach Anspruch 1, dadurch gekennzeichnet, daß der Rest X in Formel I einen Rest nach Formel III bedeutet, 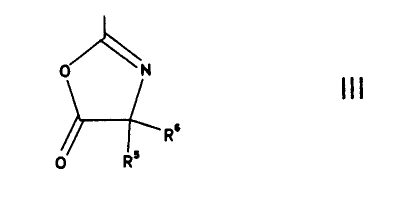 worin
R⁵ und R⁶ unabhängig voneinander
H oder Alkyl mit 1-5 C-Atomen
bedeuten.

4. Aktivierte Trägermaterialien, dadurch gekennzeichnet, daß als aktivierte Gruppierung ein Rest entsprechend einer der Formeln VIIa oder VIIb, 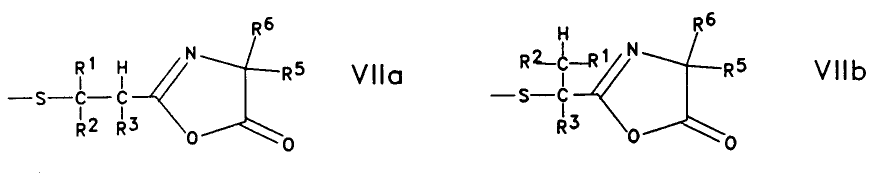 worin
R¹, R² und R³ unabhängig voneinander
H oder CH₃
und
R⁵ und R⁶ jeweils unabhängig von einander
H oder Alkyl mit 1-5 C-Atomen
bedeuten, enthalten ist.

5. Verfahren zur Herstellung von aktivierten Trägermaterialien auf Grundlage von hydroxylgruppenhaltigen Basisträgern, auf deren Oberflächen Polymere durch Pfropfpolymerisation kovalent gebunden werden, dadurch gekennzeichnet, daß die hydroxylgruppenhaltigen Basisträgerteilchen in Gegenwart von Cer(IV)-Ionen in einer Lösung enthaltend Monomere der Formel IV 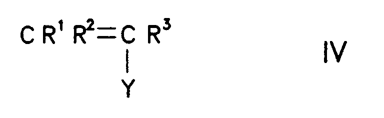 worin
R¹, R² und R³ unabhängig voneinander
H oder CH₃
und
Y eine aktivierte oder aktivierbare Gruppierung, enthaltend eine Oxiran- oder eine Azlactongruppe oder einen Rest, aus dem eine Azlactongruppe hervorgehen kann,
bedeuten,
suspendiert und polymerisiert werden, und gegebenenfalls eine Vorläuferverbindung in eine Azlactongruppe umgewandelt wird.

6. Verfahren nach Anspruch 5, dadurch gekennzeichnet, daß unterschiedliche Monomere der Formel IV copolymerisiert werden.

7. Verfahren nach einem der Ansprüche 5 oder 6, dadurch gekennzeichnet, daß zusätzlich zu Monomeren der Formel IV Monomere der Formel V copolymerisiert werden, 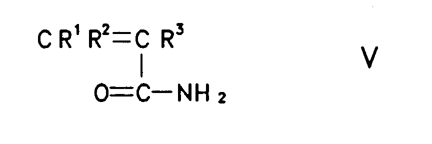 worin
R¹, R² und R³ unabhängig voneinander
H oder CH₃
bedeuten.

8. Verfahren zur Herstellung eines aktivierten Trägermaterials nach Anspruch 4, dadurch gekennzeichnet, daß man einen Basisträger, der Thiolgruppen enthält, mit einem Vinylazlacton der Formel VIII, 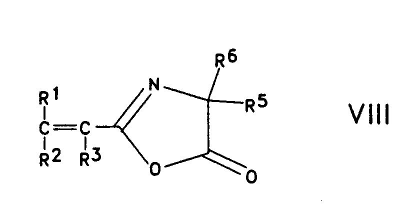 worin
R¹, R² und R³ unabhängig voneinander H oder CH₃
und
R⁵ und R⁶ jeweils unabhängig von einander
H oder Alkyl mit 1-5 C-Atomen
bedeuten, umsetzt.

9. Verwendung eines aktivierten Trägermaterials nach einem der Ansprüche 1 bis 4 für die Bindung von Affinitätsliganden.

10. Verwendung eines aktivierten Trägermaterials nach einem der Ansprüche 1 bis 4 Fur die Immobilisierung von Enzymen.

11. Verfahren zur Herstellung von Affinitätsträgern, dadurch gekennzeichnet, daß Affinitätsliganden an aktivierte Trägermaterialien nach einem der Ansprüche 1 bis 4 gebunden werden.

12. Affinitätsträger herstellbar nach einem Verfahren nach Anspruch 11.

13. Affinitätsträger nach Anspruch 12 enthaltend Protein A.

14. Verwendung von Affinitätsträgern nach einem der Ansprüche 12 bis 13 bei der Aufreinigung von Biopolymeren mittels Affinitätschromatographie.

15. Verwendung eines Affinitätsträgers nach einem der Ansprüche 12 bis 13 für Isolierung von Immunglobulinen.

16. Verfahren zur Immobilisierung eines Enzyms dadurch gekennzeichnet, daß dieses an aktivierte Trägermaterialien nach einem der Ansprüche 1 bis 4 gebunden wird.

17. Immobilisiertes Enzym herstellbar nach einem Verfahren nach Anspruch 16.

18. Immobilisierte Benzonase herstellbar nach einem Verfahren nach Anspruch 16.

19. Verwendung von immobilisierter Benzonase nach Anspruch 18 zur Hydrolyse von Nukleinsäuren.

## Claims

1. Activated support materials based on base supports containing hydroxyl groups, on the surfaces of which polymers are covalently bonded, characterized in that
a) the base support contains aliphatic hydroxyl groups,
b) the covalently bonded polymers are bonded to the support via a terminal monomer unit,
c) the polymers contain monomer units of the formula I and
d) the monomer units are linked linearly, 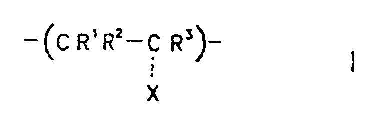 wherein
R¹, R² and R³ independently of one another are
H or CH₃
and
X is an activated grouping containing an azlactone or
an oxirane group.

2. Activated support materials according to Claim 1, characterized in that the radical X in formula I is a radical according to formula II 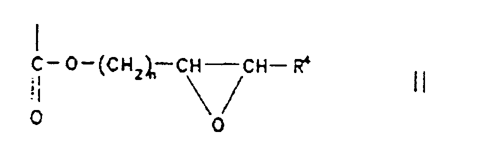 wherein
R⁴ is H, alkyl having 1-5 C atoms or aryl having 6-12 C atoms
and
n is an integer between 1 and 5.

3. Activated support materials according to Claim 1, characterized in that the radical X in formula I is a radical according to formula III 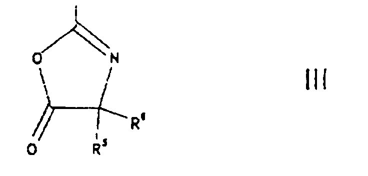 wherein
R⁵ and R⁶ independently of one another are
H or alkyl having 1-5 C atoms.

4. Activated support materials, characterized in that they contain, as the activated grouping, a radical corresponding to one of the formulae VIIa or VIIb 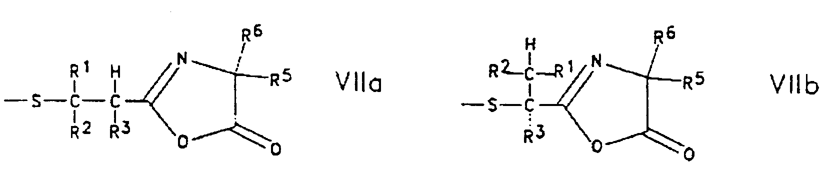 wherein
R¹, R² and R³ independently of one another are
H or CH₃
and
R⁵ and R⁶ in each case independently of one another are H
or alkyl having 1-5 C atoms.

5. Process for the preparation of activated support materials based on base supports containing hydroxyl groups, on the surfaces of which polymers are covalently bonded by grafting polymerization, characterized in that the base support particles containing hydroxyl groups are suspended and polymerized, in the presence of cerium(IV) ions, in a solution comprising monomers of the formula IV 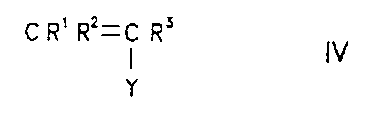 wherein
R¹, R² and R³ independently of one another are
H or CH₃
and
Y is an activated or activatable grouping containing an oxirane or an azlactone group or a radical from which an azlactone group can originate,
and, if appropriate, a precursor compound is converted into an azlactone group.

6. Process according to Claim 5, characterized in that different monomers of the formula IV are copolymerized.

7. Process according to one of Claims 5 or 6, characterized in that, in addition to monomers of the formula IV, monomers of the formula V are copolymerized 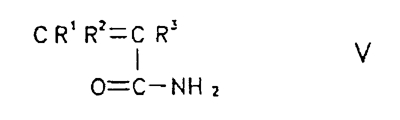 wherein
R¹, R² and R³ independently of one another are
H or CH₃.

8. Process for the preparation of an activated support material according to Claim 4, characterized in that a base support which contains thiol groups is reacted with a vinylazlactone of the formula VIII 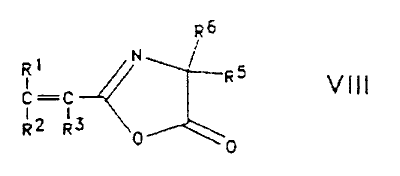 wherein
R¹, R² and R³ independently of one another are
H or CH₃
and
R⁵ and R⁶ in each case independently of one another are
H or alkyl having 1-5 C atoms.

9. Use of an activated support material according to one of Claims 1 to 4 for bonding affinity ligands.

10. Use of an activated support material according to one of Claims 1 to 4 for immobilizing enzymes.

11. Process for the preparation of affinity supports, characterized in that affinity ligands are bonded to activated support materials according to one of Claims 1 to 4.

12. Affinity support which can be prepared by a process according to Claim 11.

13. Affinity support according to Claim 12 containing protein A.

14. Use of affinity supports according to one of Claims 12 to 13 in the purification of biopolymers by means of affinity chromatography.

15. Use of an affinity support according to one of Claims 12 to 13 for isolating immunoglobulins.

16. Process for the immobilization of an enzyme, characterized in that this is bonded to activated support materials according to one of Claims 1 to 4.

17. Immobilized enzyme which can be prepared by a process according to Claim 16.

18. Immobilized benzonase which can be prepared by a process according to Claim 16.

19. Use of immobilized benzonase according to Claim 18 for the hydrolysis of nucleic acids.

## Revendications

1. Matières de support activées basées sur des supports de base contenant des groupements hydroxyle, sur les surfaces desquelles des polymères sont liés de façon covalente, caractérisées en ce que:
a) le support de base contient des groupements hydroxyle aliphatiques;
b) les polymères liés de façon covalente sont liés au support via un motif monomère terminal;
c) les polymères contiennent des motifs monomères de formule I; et
d) les motifs monomères sont liés de façon linéaire; 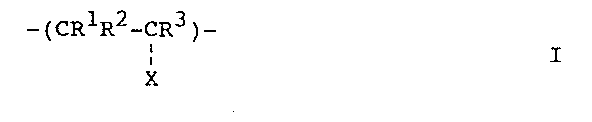 dans lesquels:
R¹, R² et R³, indépendamment l'un de l'autre, sont H ou CH₃
et
X est un groupement activé contenant une azlactone ou un groupement oxirane.

2. Matières supports activées selon la revendication 1, caractérisées en ce que le radical X dans la formule I est un radical selon la formule II: 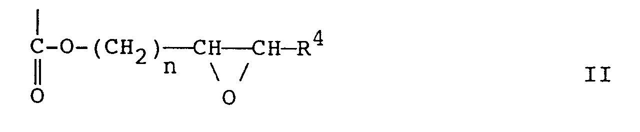 dans laquelle:
R⁴ est H, un groupement alkyle ayant 1 à 5 atomes de carbone ou aryle ayant 6 à 12 atomes de carbone
et
n est un nombre entier compris entre 1 et 5.

3. Matières support activées selon la revendication 1, caractérisées en ce que le radical X dans la formule I est un radical selon la formule III 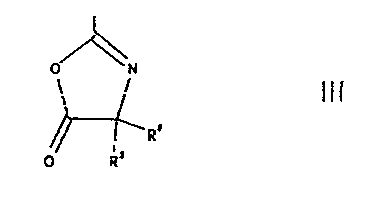 dans laquelle
R⁵ et R⁶, indépendamment l'un de l'autre, sont H ou un
groupement alkyle ayant 1 à 5 atomes de carbone.

4. Matières support activées, caractérisées en ce qu'elles contiennent, en tant que groupement activé, un radical correspondant à une des formules VIIa ou VIIb 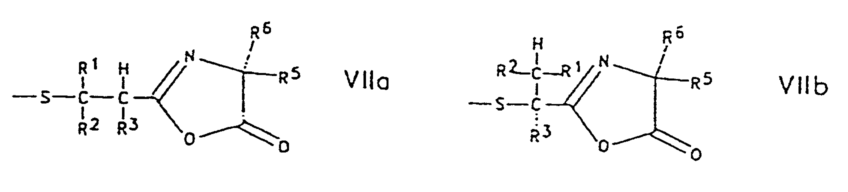 dans lesquelles
R¹, R² et R³, indépendamment l'un de l'autre, sont H ou CH₃
et
R⁵ et R⁶ dans chaque cas, indépendamment l'un de l'autre,
sont H ou un groupement alkyle ayant 1 à 5 atomes de carbone.

5. Procédé pour la préparation de matières support activées basées sur les supports de base contenant des groupements hydroxyle, sur les surfaces desquels des polymères sont liés de façon covalente au moyen d'une polymérisation par greffage, caractérisé en ce que les particules support de base contenant les groupements hydroxyle sont mises en suspension ou polymérisées, en présence d'ions cérium (IV), dans une solution comprenant des monomères de formule IV 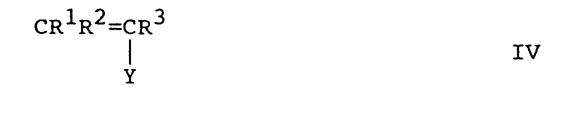 dans laquelle:
R¹, R² et R³, indépendamment l'un de l'autre, sont H ou CH₃
et
Y est un groupement activé ou activable contenant un groupement oxirane ou un groupement azlactone ou un radical pouvant être à l'origine d'un groupement azlactone,
et, si approprié, un composé précurseur est converti en un groupement azlactone.

6. Procédé selon la revendication 5, caractérisé en ce que les différents monomères de formule IV sont copolymérisés.

7. Procédé selon l'une quelconque des revendications 5 ou 6, caractérisé en ce que, outre les monomères de formule IV, les monomères de formule V sont copolymérisés 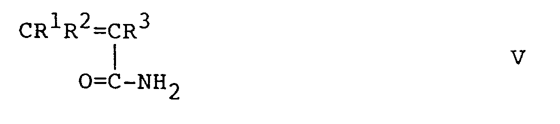 dans laquelle
R¹, R² et R³, indépendamment l'un de l'autre, sont H ou CH₃.

8. Procédé pour la préparation d'une matière support activée selon la revendication 4, caractérisée en ce qu'un support de base qui contient les groupements thiols est mis à réagir avec une vinylazlactone de formule VIII 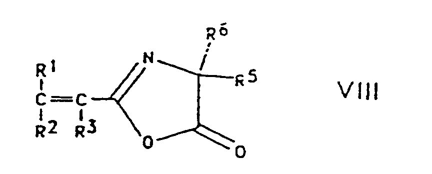 dans laquelle
R¹, R² et R³, indépendamment l'un de l'autre, sont H ou CH₃
et
R⁵ et R⁶, dans chaque cas indépendamment l'un de l'autre,
sont H ou un groupement alkyle ayant 1 à 5 atomes de carbone.

9. Utilisation d'une matière support activée selon l'une quelconque des revendications 1 à 4 pour lier des ligands par affinité.

10. Utilisation d'une matière support activée selon l'une quelconque des revendications 1 à 4 pour immobiliser des enzymes.

11. Procédé pour la préparation de supports d'affinité, caractérisé en ce que les ligands d'affinité sont liés aux matières support activées selon l'une quelconque des revendications 1 à 4.

12. Support d'affinité que l'on peut préparer à l'aide d'un procédé selon la revendication 11.

13. Support d'affinité selon la revendication 12 contenant une protéine A.

14. Utilisation de supports d'affinité selon l'une quelconque des revendications 12 et 13 dans la purification des biopolymères à l'aide d'une chromatographie par affinité.

15. Utilisation d'un support d'affinité selon l'une quelconque des revendications 12 et 13 pour isoler les immunoglobulines.

16. Procédé pour l'immobilisation d'une enzyme, caractérisé en ce que celle-ci est liée à des matières support activées selon l'une quelconque des revendications 1 à 4.

17. Enzyme immobilisée que l'on peut préparer à l'aide d'un procédé selon la revendication 16.

18. Benzonase immobilisée que l'on peut préparer à l'aide d'un procédé selon la revendication 16.

19. Utilisation de benzonase immobilisée selon la revendication 18 pour l'hydrolyse d'acides nucléiques.
